(19) Europäisches Patentamt
European Patent Office
Office européen des brevets

(11) Numéro de publication : **0 323 332 B1**

# (12) FASCICULE DE BREVET EUROPEEN

(45) Date de publication du fascicule du brevet : **27.11.91 Bulletin 91/48**

(21) Numéro de dépôt : **88403300.2**

(22) Date de dépôt : **23.12.88**

(51) Int. Cl.[5] : **C07C 29/40**, C07F 13/00, C07C 33/025, C07C 33/14, C07C 33/30, C07C 33/48, C07C 67/28, C07C 67/343, C07C 69/73

## (54) Perfectionnement à la production d'alcools tertiaires et secondaires par l'action d'un composé organique halogéné et du manganèse sur un composé porteur d'un groupe carbonyle.

(30) Priorité : **30.12.87 FR 8718352**

(43) Date de publication de la demande : **05.07.89 Bulletin 89/27**

(45) Mention de la délivrance du brevet : **27.11.91 Bulletin 91/48**

(84) Etats contractants désignés : **BE CH DE GB IT LI NL**

(56) Documents cités :
**EP-A- 0 029 665**
**US-A- 4 298 542**
**TETRAHEDRON LETTERS, no. 38, 1977, pp. 3383-3384, Pergamon Press, Oxford, GB; E. CAHIEZ et al, "Reactivity of organomanganese (II) reagents. Reaction with carbonyl compounds, selective preparation of ketols from keto-aldehydes"**
**ORGANOMETALLICS, no. 1, 1982, pp. 1249-1251, American Chemical Society, Washington D.C., US; T. HIYAMA et al, "Grignard-type carbonyl adddition of allyl units with MnC12-LiAlH4 reagent"**

(56) Documents cités :
**CHEMISTRY LETTERS, 1983, pp. 1237-1238, Chemical Society of Japan; T. HIYAMA et al, "Carbon-carbon bond formation with metallic manganese"**
**L'ACTUALITE CHIMIQUE, septembre 1984, pages 24-28, Paris, FR; G. CAHIEZ, "Les organomanganeux: utilisations en synthèse organique", page 27, colonne 2, ligne 11 du fond**

(73) Titulaire : **SOCIETE NATIONALE ELF AQUITAINE**
**Tour Elf, 2, Place de la Coupole, La Défense 6**
**F-92400 Courbevoie (FR)**

(72) Inventeur : **Cahiez, Gérard**
**68 rue de Turbigo**
**F-75003 Paris (FR)**
Inventeur : **Chavant, Pierre-Yves**
**70 rue Dutot**
**F-75015 Paris (FR)**
Inventeur : **Tozzolino, Pierre**
**Chemin Carrerot Serres-Morlaas**
**F-64160 Morlaas (FR)**

(74) Mandataire : **Bertrand, Didier et al**
**Cabinet FEDIT-LORIOT 38, Avenue Hoche**
**F-75008 Paris (FR)**

EP 0 323 332 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un perfectionnement à la préparation d'alcools secondaires et tertiaires. Elle se rapporte plus spécialement à l'obtention de tels alcools par l'action d'un composé organo-manganeux sur un composé porteur d'un groupe carbonyle.

L'utilité des alcools secondaires et tertiaires est bien connue dans la synthèse de produits naturels ; parmi les méthodes classiques de leur préparation, celles qui sont basées sur l'emploi de réactifs organométalliques sont très intéressantes, parce qu'elles permettent d'obtenir des types variés d'alcools tertiaires, utiles notamment dans le domaine pharmaceutique, ainsi que dans celui des aromes et parfums. La voie par composés organométalliques a fait l'objet de nombreux travaux, comme, entre autres, ceux de BARBIER ("C.R. Acad. Sci. Paris vol. 128, p. 110, 1899) qui décrivent la préparation d'alcools à partir d'halogénures organiques, de magnésium et de composés carbonylés. Cette réaction est une modification de celle de WAGNER et SAYTZEEF ("Justus Liebigs Ann. Chem. vol. 175, p. 351, 1875), utilisant le zinc, et se trouvant à l'origine du remarquable développement de la chimie des organomagnésiens. L'utilisation du zinc a fait l'objet de travaux importants ; il s'agit notamment de la réaction d'un $\alpha$-bromoester et d'un composé carbonylé en présence de zinc. Cette réaction est communément appelée réaction de Reformatsky.

Des travaux récents décrivent ce même type de réaction à partir de divers métaux tels que l'étain, le cérium ou les couples Zn/Cu, Zn/Cd, Zn/Pb dans la réaction de Reformatsky.

Un progrès très net a été réalisé dans le domaine des organo-métalliques par l'emploi d'organo-manganeux, dont l'avantage réside dans leur sélectivité lors de l'attaque de molécules multifonctionnelles. Ainsi, selon G. CAHIEZ ("L'Actualité Chimique", Septembre 1984, p. 27) les organo-manganeux réagissent, comme de nombreux organo-métalliques, avec les aldéhydes et les cétones pour conduire aux alcools tertiaires, mais ils n'attaquent pas les esters. Cette sélectivité est intéressante puisqu'elle est totale, même à température ambiante.

Jusqu'à une période récente, les dérivés organo-manganeux étaient obtenus par échange métal-métal à partir des lithiens et magnésiens. Cette méthode de préparation interdit l'accès à des organo-manganeux fonctionnels tel que X-Mn-$CH_2$-$CO_2$Et uniquement parce que le lithien ou magnésien de départ ne peut être obtenu. Cela met en évidence l'avantage qui consiste à préparer les composés organo-manganeux directement à partir du manganèse métallique et d'un halogénure organique. Récemment, HIYAMA et coll. (Organometallics 1982, 1, 1249-1251) décrivent la réactivité du manganèse métal obtenu par réduction à l'aide d'hydrure $LiAlH_4$ du chlorure de manganèse (II), vis-à-vis de bromures allyliques ; le réactif obtenu, traité par un aldéhyde ou une cétone, conduit à l'alcool tertiaire correspondant. Selon une publication ultérieure (Chemistry Letters, p. 1237-38, 1983), l'utilisation de manganèse micronisé est économiquement plus intéressante. Cependant, l'obtention de bons rendements nécessite la mise en oeuvre de quantités excessives de réactifs : rapports métal/cétone ou aldéhyde = 7/1, halogénure/cétone ou aldéhyde = 6. De plus, la réaction exige 1 éq. d'iode pour un reflux pendant une douzaine d'heures. Il s'agit donc d'un ensemble de conditions qui rendent l'exploitation industrielle de telles synthèses très hypothétique.

La présente invention apporte une solution nouvelle qui permet de produire toutes sortes d'alcools secondaires ou tertiaires par l'action d'un halogénure organique, de manganèse métallique et d'un composé organique porteur d'un carbonyle à des températures voisines de l'ambiante, et dans des bonnes conditions économiques. L'invention permet, en effet, de produire les alcools voulus bien plus vite et avec des rendements meilleurs qu'on ne pouvait le faire selon la technique antérieure.

Le procédé suivant l'invention, qui consiste à faire réagir un halogénure organique avec un composé organique, porteur d'au moins un groupe carbonyle, au sein d'un solvant organique, en présence de manganèse métallique en poudre et à hydrolyser ensuite le produit formé, est caractérisé en ce que la réaction avec le manganèse est initiée et activée par la présence d'un ester.

Ainsi accélère-t-on la réaction et augmente-t-on son rendement par l'emploi d'un ester, dans la solution organique ou dans la suspension des substances susindiquées. L'invention peut être réalisée par l'adjonction d'ester approprié au solvant utilisé ; lorsque les réactifs sont solubles dans cet ester, la forme d'exécution préférée de l'invention consiste à employer l'ester lui-même comme liquide du milieu réactionnel.

L'amélioration dans la production d'alcools suivant l'invention est obtenue plus particulièrement à l'aide des esters de relativement faible masse moléculaire, notamment de ceux dont le nombre total d'atomes de carbone est de 3 à 13. Autrement dit, l'acide carboxylique de l'ester comporte 2 à 12 C et son reste d'alcool est en $C_{12}$ à $C_1$ respectivement. Ainsi peut-on employer les esters des acides tels que acétique, propionique, butyrique, caproïque, benzoïque, etc., de méthyle, éthyle, propyle, butyle, octyle ou autre. Cependant, des résultats particulièrement intéressants sont obtenus avec les solvants fort économiques que constituent les acides aliphatiques en $C_2$ à $C_4$ avec des alcools en $C_1$ à $C_3$ : tels sont les acétates, propionates, butyrates et iso-butyrates de méthyle, éthyle, propyle ou isopropyle. Economiquement, l'acétate d'éthyle constitue un solvant de choix pour la réalisation de l'invention.

Selon une forme d'exécution particulière de l'invention on obtient une amélioration également dans la production d'alcools secondaires, ainsi que dans celle des alcools tertiaires : cette forme d'exécution réside dans l'adjonction au milieu réactionnel d'un composé de métal moins électropositif que le manganèse, en même temps qu'un ester. Ainsi, active-t-on la réaction par l'addition au milieu, dans le solvant organique constitué par ou contenant un ester, d'un sel tel qu'halogénure, sulfate, acétate ou autre, d'un métal de groupe II à VIII de la Classification Périodique des éléments, moins électropositif que Mn.

Conviennent particulièrement des composés de métaux Zn, Cd, Sn et Hg et surtout de Zn. La quantité de composé métallique est en général de 1 à 200% d'équivalent par litre de solution formant le milieu réactionnel et de préférence de 2 à 10%.

Lorsque le composé métallique est présent dans le milieu réactionnel conjointement avec un ou plusieurs des esters décrits plus haut, le composé organique à groupe carbonyle peut être un aldéhyde, et l'on obtient alors un alcool secondaire. La production d'alcools tertiaires à partir de cétones se fait avec des rendements encore meilleurs que lors de l'emploi d'un ester seul, sans composé métallique.

Les solvants classiques, connus pour leur emploi dans la technique antérieure du procédé de l'invention, notamment des éthers et, éventuellement hydrocarbures aliphatiques ou aromatiques ou solvants chlorés, peuvent être employés, additionnés d'ester suivant l'invention. En général,à raison de 5 à 90% en poids, de préférence de 50 à 90%. On peut, de cette façon, dans la mesure où les produits de la réaction sont partiellement solubles, employer, par exemple, THF, diéthyléther, heptane, octane, benzène, toluène, acétonitrile, DMF, trichloroéthane, etc. additionné d'une proportion compatible d'ester.

Comme indiqué plus haut, il est en général fort avantageux d'utiliser un ou plusieurs esters eux-mêmes en tant que solvant de la réaction.

L'ensemble des réactions, qui forment le procédé de l'invention, peut être représenté de la façon suivante.

(1) 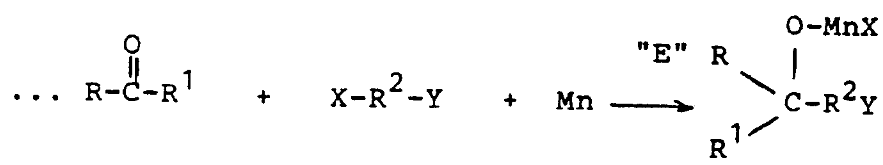


(A) (B) (C)

(2) 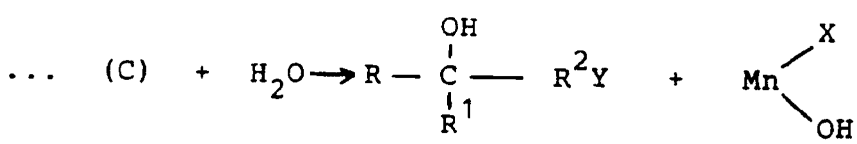

(D)

La réaction (1) a lieu au sein d'un solvant, dans lequel sont de préférence solubles les deux réactifs (A) et (B). Le principe de l'invention réside en l'addition d'un ester activant "E".

L'organo-manganeux (C) peut être séparé du milieu réactionnel, pour être soumis à l'hydrolyse (2) ; on peut notamment évaporer le solvant après la réaction (1), et traiter le résidu à l'eau à la manière connue en soi. L'hydrolyse peut aussi avoir lieu directement, dans le solvant, après la réaction (1). Elle est en général effectuée en milieu acide, et le Mn est récupéré sous la forme de son sel $MnX_2$.

Le composé (A) est un aldéhyde lorsque $R^1$ est un H, ou une cétone quand R et $R^1$ sont des groupes hydrocarbonés ; ceux-ci peuvent être aliphatiques, de préférence en $C_1$ à $C_8$, cycloaliphatiques surtout en $C_4$ à $C_8$, ou/et aryliques, notamment phényles ou naphtyles pouvant porter des substituants alkyliques, alcényliques, halogènes ou autres.

R, dans le cas d'aldéhyde, et R ou/et $R^1$ dans celui de cétone, peuvent porter des fonctions telles que ester, nitrile, éther, sulfure, halogénure, acétal. Ainsi, le réactif (A) peut être par exemple tel qu'acétaldéhyde, propionaldéhyde, butyraldéhyde, phénylacétaldéhyde, benzaldéhyde, aldéhyde cinnamique, anisaldéhyde, etc. Il peut être constitué par une cétone du type acétone, dipropylcétone, méthyléthylcétone, méthylheptènone, cyclopentanone, méthylcyclohexanone, acétylpropionate de méthyle, acétophénone, benzophénone, menthone, naphtylméthylcétone, etc.

Dans l'halogénure organique (B), la lettre X désigne un halogène, surtout Br. $R^2$ désigne un groupe hydrocarboné. Y, dont la présence n'est pas obligatoire, représente un groupe fonctionnel, tel que par exemple

—COOH, nitrile, amide, ou groupe à double ou triple liaison. L'halogénure organique peut être allylique, propargylique, benzylique ou un α-halogénoester ou nitrile. Ainsi, le composé X-$R^2$-Y peut être par exemple : Br-$CH_2$-CH=$CH_2$ · Cl-$CH_2$-CH=$CH_2$ ; Br-$CH_2C_6H_5$ ; Br-$CH_2$-C($CH_3$)=$CH_2$ ; Br-$CH_2$-$CO_2$Et ; I-$CH_2$-CH=C$(CH_3)_2$.

Les corps, cités plus haut, ne constituent que des exemples non limitatifs des composés (A) et (B), donnés seulement pour fixer les idées.

Le procédé suivant l'invention peut être réalisé avec des rapports stoechiométriques des réactifs (A), (B) et Mn dans la réaction (1). Toutefois, il s'avère préférable d'opérer avec un certain excès de métal Mn et de composé (B) par rapport au composé carbonylé (A). En effet, les rendements en l'alcool secondaire ou tertiaire sont accrus lorsque Mn et (B) sont en excès par rapport à la stoechiométrie. De façon générale, il est bon d'employer 1 à 3 atomes de Mn et 1 à 2 moles de (B) par mole de composé carbonylé (A). Les proportions préférées se situent entre 1,2 et 1,6 atomes Mn et 1,1 à 1,5 moles d'halogénure (B).

D'autre part, il est avantageux de travailler avec des solutions assez mais pas trop concentrées en composé (A), notamment solutions 0,3 à 2 M et de préférence 0,5 à 1,6 M.

La température durant l'étape (1) du procédé peut être comprise entre 20° et 100°C, la marge de 30° à 60°C étant préférable.

La granulométrie du manganèse employé est de 1 à 2000 microns et de préférence de 10 à 500 microns. Il est même possible, pour des réactions à plus grande échelle, d'utiliser des granulométries moyennes supérieures à 2000 microns.

L'addition des réactifs doit être effectuée pendant un temps qui est en général de l'ordre de 1 à 6 heures, et très souvent de 3 1/2 à 4 1/2 heures ; cela constitue un avantage marqué sur la technique antérieure qui exigeait 12 heures ou plus de temps de réaction. Grâce à l'addition de l'ester activateur, la réaction se déclenche au bout de 30 à 40 minutes, alors qu' elle commence seulement au bout d'environ 2 heures dans le procédé connu à la poudre de manganèse.

Par rapport au procédé décrit ci-dessus, l'addition de sels de Zn, Cd, Hg, Sn, etc., solubles au moins partiellement dans le milieu réactionnel, et notamment d'halogénures, à raison de 1 à 200% par rapport au composé carbonylé et de préférence de 2 à 10%, permet d'améliorer considérablement certains rendements. Il en est ainsi pour les halogénures dans le cas du chlorure d'allyle et des α-chloroesters et lorsque le composé carbonylé (A) est un aldéhyde RCHO ou une cétone méthylée $RCOCH_3$, R étant défini comme précédemment.

L'invention est illustrée par des exemples non limitatifs, donnés plus loin, dans lesquels le mode opératoire que voici était appliqué.

Le réacteur était constitué par un tricol de 1000 ml de contenance, muni d'un agitateur mécanique, monté de façon à mettre en mouvement la poudre de Mn que l'on plaçait dans le réacteur. Celui-ci comportait également un thermomètre et une tubulure d'amenée d'azote permettant d'assurer une atmosphère inerte, dans l'appareil, tout au long des opérations. Le réacteur était placé dans un bain d'eau maintenu à la température voulue.

Le manganèse était à 98-99% en poudre grossière d'une granulométrie comprise entre 10 et 500 microns. Il était chargé dans le tricol, avec activateur, et recouvert de solvant. La réaction était initiée par quelques gouttes d'halogénure organique (B), X$R^2$Y, ou de bromure d'allyle : dès que le métal a subi un changement de coloration, et qu'un échauffement s'est produit, on commençait à introduire progressivement à l'aide de pompes les réactifs (A) et (B) en solution dans l'acétate d'éthyle. A la fin de cette introduction, qui durait quelques heures, on continuait à agiter le milieu durant 15 à 30 mn à la même température, à laquelle a été opérée la réaction (1).

On mélangeait ensuite le milieu réactionnel avec approximativement son volume d'eau, à 20°C, légèrement acidifiée avec de l'HCl, de façon à amener le pH de l'eau à 7 ou à une valeur un peu inférieure, pour dissoudre les sels métalliques présents.

Le produit recherché était extrait à l'éther et lavé au bicarbonate de Na.

Le rendement en l'alcool obtenu, par rapport au composé (A), était calculé à partir de la quantité d'alcool isolé par extraction ou déterminée par chromatographie en phase vapeur.

## EXEMPLES 1 à 3

### Préparation de l'allyl-4 heptanol-4 comparativement selon la technique antérieure et suivant l'invention

En opérant comme indiqué plus haut, on fait réagir 1 mole de di-n-propyl cétone $CH_3CH_2CH_2$-CO-$CH_2CH_2$-$CH_3$ avec 1 mole de bromure d'allyle $CH_2$=CH-$CH_2$Br et 1,5 atomes de Mn métallique en poudre, dans du tétrahydrofurane bien sec, à la proportion 0,7 litre de ce solvant pour les quantités susindiquées. La réaction a lieu à 50°C, pendant 4 heures.

EXEMPLE 1

Rien d'autre n'est ajouté au milieu réactionnel. La réaction ne peut être initiée. Elle se déclenche au cours de l'addition des réactifs et conduit au bout de 4 heures à un rendement en alcool tertiaire de 16% seulement.

EXEMPLE 2

Dans le mélange réactionnel décrit plus haut, on a remplacé 50% de THF par de l'acétate d'éthyle sec. La réaction se déclenche en quelques minutes après introduction des réactifs. Le rendement au bout de 4 heures à 50°C, en alcool tertiaire est de 58% par rapport à la dipropylcétone. L'activation par l'acétate d'éthyle est donc très net.

EXEMPLE 3

Dans le mélange réactionnel des essais précédents, le solvant THF est remplacé complètement par le même volume d'acétate d'éthyle sec. On trouve alors, après 4 heures à 50°C, un rendement en alcool tertiaire de 73%. L'effet de l'ester est donc remarquable, par comparaison avec l'exemple 1.

EXEMPLE 4

Rôle de la température

L'exemple 3 est répété à quatre températures différentes pour le premier stade de la préparation, la concentration de la dipropyl cétone étant de 1,33 M dans l'acétate d'éthyle. Les rendements sur la cétone, déterminés par chromatographie, sont :

pour 20°C ................. 54 %
pour 40°C ................. 61%
" 50°C ................. 73%
" 60°C ................. 67%

EXEMPLE 5

Influence des proportions de réactifs

Des préparations identiques à celles de l'exemple 3 sont effectuées avec des proportions variables de bromure (B) et de Mn. Elles ont conduit aux rendements suivants, indiqués par la chromatographie en phase vapeur.

| Moles de $CH_2$=CH-$CH_2$Br par mole de cétone | Atomes Mn par mole cétone | Rendement % |
|---|---|---|
| 1,0 | 1,5 | 72 |
| 1,0 | 2,5 | 72 |
| 1,5 | 1,5 | 92 |
| 1,5 | 2,5 | 98 |
| 1,8 | 1,5 | 94 |
| 2,0 | 2,5 | 100 |

Les excès de réactifs permettent d'atteindre des rendements très élevés.

EXEMPLE 6

Effet de la concentration en cétone

En répétant l'exemple 3 avec des concentrations molaires variables de dipropyl cétone, avec 1,3 mole d'halogénure et 1,5 atome Mn, on a trouvé les rendements suivants.

| Concentration de cétone dans le milieu réactionnel | Rendement % |
|---|---|
| 0,83 M | 83 |
| 1,67 | 92 |

Il existe donc un certain optimum.

EXEMPLES 7 à 23

Préparation de différents alcools tertiaires à partir de diverses cétones R-CO-$R^1$ avec 1,4 mole de bromure d'alcényle $CH_2$=CH-$CH_2$Br et 1,5 atome de Mn, à 50°C, durant 4 heures. Les rendements par rapport à la cétone sont donnés dans le tableau I, à la page suivante.

## TABLEAU I

| N° | R-CO-R[1] | Rendement % |
|---|---|---|
| 7 | $CH_3CH_2CH_2-\underset{\underset{O}{\|}}{C}-CH_2CH_2CH_3$ | 85 |
| 8 | $CH_3(CH_2)_3-\underset{\underset{O}{\|}}{C}-(CH_2)_3CH_3$ | 86 |
| 9 | $CH_3(CH_2)_4-\underset{\underset{O}{\|}}{C}-(CH_2)_4CH_3$ | 74 |
| 10 | $CH_3(CH_2)_6-\underset{\underset{O}{\|}}{C}-(CH_2)_6CH_3$ | 81 |
| 11 | $C_6H_5-\underset{\underset{O}{\|}}{C}-CH_2CH_2CH_3$ | 91 |
| 12 | $p.Cl-C_6H_4-\underset{\underset{O}{\|}}{C}-CH_2CH_3$ | 91 |
| 13 | $C_6H_5-\underset{\underset{O}{\|}}{C}-CH_3$ | 78 |
| 14 | $p.CH_3-C_6H_4-\underset{\underset{O}{\|}}{C}-CH_3$ | 72 |
| 15 | $p.Br-C_6H_4-\underset{\underset{O}{\|}}{C}-CH_3$ | 85 |
| 16 | 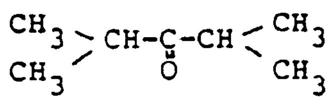 | 68 |
| 17 | 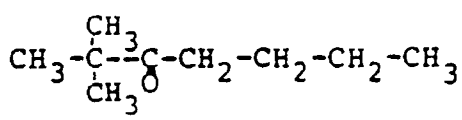 | 62 |
| 18 | 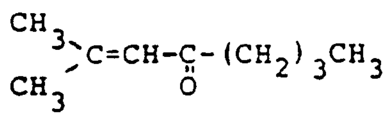 | 75 |
| 19 | 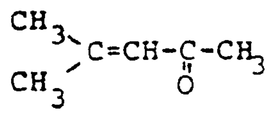 | 59 |
| 20 | $C_6H_{10}CO$ (cyclohexanone) | 56 |
| 21 | $CH_3(CH_2)_3-\underset{\underset{O}{\|}}{C}-(CH_2)_6COOC_2H_5$ | 87 |
| 22 | $C_2H_5-\underset{\underset{O}{\|}}{C}-(CH_2)_{10}-O-\underset{\underset{O}{\|}}{C}-CH_3$ | 74 |
| 23 | $C_2H_5-\underset{\underset{O}{\|}}{C}-(CH_2)_5Cl$ | 79 |

Selon le mode opératoire décrit plus haut, avant les exemples, mais en ajoutant 0,1 mole de chlorure de zinc par mole de composé carbonylé, avant l'étape d'initiation de la réaction, on obtient de bons rendements, en utilisant comme dérivé carbonylé un aldéhyde. Les rendements obtenus dans le cas des cétones méthylées sont également améliorés.

EXEMPLE 24

Selon les conditions opératoires décrites plus haut, sans addition de sel de zinc, en utilisant 1 mole d'heptanal, 1,1 moles de bromure d'allyle, 1,3 atome-gramme de manganèse et 0,7 litre d'acétate d'éthyle, on obtient, après 4 heures, un rendement de 5% en alcool II, récupéré sous forme d'acétate.

EXEMPLE 25

Le même essai qu'à l'exemple 24, conduit avec 0,1 mole de chlorure de zinc, fournit au bout de 4 heures, un rendement de 85% en alcool II, récupéré sous forme d'acétate. L'effet du chlorure de zinc est donc remarquable.

EXEMPLE 26

On opère selon la même procédure que pour l'exemple 25, en présence de 0,05 mole de chlorure de zinc, à partir de 1 mole d'heptylméthylcétone, 1,3 atome-gramme de manganèse, 1,1 mole de bromure de crotyle et 0,7 litre d'acétate d'éthyle. Le rendement en alcool tertiaire est de 80% par rapport à la cétone.

EXEMPLE 27

Dans les conditions de l'exemple 25, en utilisant 0,1 mole de chlorure de zinc, 1,1 mole de bromoacétate d'éthyle, 1 mole d'heptanal, 1,3 atome-gramme de manganèse et 0,7 litre d'acétate d'éthyle, on obtient 60% de rendement en hydroxyester par rapport à l'heptanal.

EXEMPLES 28 à 31

Préparation de divers alcools secondaires, sous forme d'acétate, à partir de 1 mole d'aldéhyde, 1,1 à 1,4 moles d'halogénure allylique, 1,3 atome-gramme de manganèse et 0,1 mole de chlorure de zinc à 50-60°C durant 4 heures. Les rendements sont indiqués dans le tableau II :

TABLEAU II

| Ex.n° | Aldéhyde | Halogénure (rapport) | Rendement % |
|---|---|---|---|
| 28 | $(CH_3)_3C-CHO$ | $BrCH_2CH=CH_2$ (1,1) | 87 |
| 29 | $C_6H_5CHO$ | " (1,4) | 90 |
| 30 | $CH_3(CH_2)_5CHO$ | $BrCH_2C(CH_3)=CH_2$ (1,1) | 83 |
| 31 | " | $BrCH_2CH=C(CH_3)_2$ (1,1) | 79 |

EXEMPLES 32 à 38

Les opérations de l'exemple 27 ont été répétées avec différents halogénures organiques et différents esters employés comme solvants. 1,4 moles d'halogénure et 1,5 atome-gramme de manganèse sont employés chaque fois pour 1 mole d'heptanal. On opère toujours en présence de 1,1 mole d'anhydride acétique. Les rendements obtenus, en acétate de l'alcool secondaire formé, sont rassemblés dans le Tableau III :

TABLEAU III

| Ex.N° | Halogénure | Solvant | Rendement |
|---|---|---|---|
| 32 | $ClCH_2CO_2Et$ | AcOEt | 50% |
| 33 | $BrCH_2CO_2Et$ | AcOEt | 86% |
| 34 | $C_6H_{13}CHBrCO_2Et$ | AcOEt | 92% |
| 35 | " | $C_7H_{15}CO_2Et$ | 78% |
| 36 | $(CH_3)_2CHBrCO_2Et$ | AcOEt | 87% |
| 37 | " | Pivalate Et | 71% |
| 38 | $C_6H_5CH_2Br$ | AcOEt | 41% |

**Revendications**

1. Procédé de préparation d'alcools par la réaction d'un halogénure organique avec un composé carbonylé, au sein d'un solvant organique, en présence de manganèse métallique, puis hydrolyse du produit de la réaction, caractérisé en ce que la réaction est initiée et activée par un ester présent dans le milieu réactionnel.

2. Procédé suivant la revendication 1, caractérisé en ce que le nombre total d'atomes de carbone dans l'ester est de 3 à 13, l'acide carboxylique de cet ester étant en $C_2$ à $C_{12}$ et le reste d'alcool en respectivement $C_{12}$ à $C_1$.

3. Procédé selon la revendication 2, caractérisé en ce que l'ester provient d'un acide aliphatique en $C_2$ à $C_4$ et d'un alcool en $C_1$ à $C_3$.

4. Procédé suivant une des revendications 1 à 3, caractérisé en ce que l'ester est un propionate, butyrate ou isobutyrate de méthyle, éthyle, propyle ou isopropyle.

5. Procédé suivant une des revendications 1 à 3, caractérisé en ce que l'ester est l'acétate d'éthyle.

6. Procédé suivant une des revendications précédentes, caractérisé en ce que le solvant utilisé est un solvant classique additionné de 5 à 90%, et de préférence 50 à 90% en poids d'ester.

7. Procédé suivant une des revendications 1 à 5, caractérisé en ce que le solvant est entièrement constitué par de l'ester.

8. Procédé suivant une des revendications 1 à 7, caractérisé en ce que l'on fait réagir 1 mole de cétone avec 1 à 2 moles d'halogénure organique et 1 à 3 atome-grammes de manganèse, la concentration préférée en cétone, dans le milieu réactionnel, étant de 0,3 à 2 M et particulièrement de 0,5 à 1,6 M.

9. Procédé suivant une des revendications précédentes, caractérisé en ce que le milieu réactionnel contient un composé d'un métal de groupe II à VIII de la Classification Périodique des éléments, moins électropositif que le manganèse.

10. Procédé selon la revendication 9, caractérisé en ce que le composé métallique est un sel de zinc, cadmium, étain ou mercure, dissous au moins partiellement dans le milieu réactionnel, principalement un halogène, en particulier chlorure, bromure ou iodure, et notamment un sel de zinc, de préférence le chlorure.

11. Procédé suivant la revendication 9 ou 10, caractérisé en ce que l'on fait réagir 1 mole d'aldéhyde ou de cétone avec 1 à 2 moles d'halogénure organique, 1 à 3 atome-grammes de manganèse et 0,01 à 1 mole de préférence 0,05 à 0,1 mole de sel métallique,la concentration préférée en composé carbonylé, dans le milieu réactionnel, étant de 0,3 à 2 M et particulièrement de 0,5 à 1,6 M.

12. Procédé suivant une des revendications précédentes, caractérisé en ce que la réaction entre le composé carbonylé, l'halogénure organique et le manganèse a lieu entre 20° et 100°C et de préférence entre 30° et 60°C.

13. Procédé suivant une des revendications précédentes, caractérisé en ce que le composé carbonylé est une cétone du type $R\text{-}CO\text{-}R^1$ où R et $R^1$ sont des groupes hydrocarbonés aliphatiques, de préférence en $C_1$ à $C_8$, cyclo-aliphatiques, surtout en $C_4$ à $C_8$, ou aryliques, notamment phényles ou naphtyles, pouvant porter des substituants alkyliques, alcéniques ou divers groupes fonctionnels, en particulier ester, amide, nitrile ou halogénure.

14. Procédé suivant une des revendications 9 à 12, caractérisé en ce que le composé carbonylé est un aldéhyde du type R-CHO, R ayant la même signification que selon la revendication 13.

15. Procédé suivant une des revendications précédentes, caractérisé en ce que l'halogénure organique

est un composé X-$R^2$-Y, X étant un halogène, $R^2$ représentant un groupe hydrocarboné et Y, dont la présence n'est pas obligatoire, est un groupe fonctionnel ou un groupe à double ou triple liaison.

## Patentansprüche

1. Verfahren zur Herstellung von Alkoholen durch Umsetzung eines organischen Halogenids mit einer Carbonylverbindung in einem organischen Lösungsmittel in Gegenwart von metallischem Mangan, dadurch gekennzeichnet, daß die Umsetzung durch einen im Reaktionsmedium vorhandenen Ester initiiert und aktiviert wird.

2. Verfahren nach Anspruch 1, dadurch gekennzeichnet, daß die Gesamtzahl der Kohlenstoffatome im Ester 3 bis 13 ist, die Carbonsäure dieses Esters $C_2$ bis $C_{12}$ ist und der alkoholische Rest entsprechend $C_{12}$ bis $C_1$.

3. Verfahren nach Anspruch 2, dadurch gekennzeichnet, daß der Ester von einer aliphatischen $C_2$- bis $C_4$-Säure und einem $C_1$-bis $C_3$-Alkohol stammt.

4. Verfahren nach einem der Ansprüche 1 bis 3, dadurch gekennzeichnet, daß der Ester ein Methyl-, Ethyl-, Propyl- oder Isopropylpropionat, -butyrat oder -isobutyrat ist.

5. Verfahren nach einem der Ansprüche 1 bis 3, dadurch gekennzeichnet, daß der Ester Ethylacetat ist.

6. Verfahren nach einem der vorstehenden Ansprüche, dadurch gekennzeichnet, daß das verwandte Lösungsmittel ein klassisches Lösungsmittel ist, dem 5 bis 90 Gew.-%, vorzugsweise 50 bis 90 Gew.-%, Ester zugesetzt wird.

7. Verfahren nach einem der Ansprüche 1 bis 5, dadurch gekennzeichnet, daß das Lösungsmittel vollständig vom Ester gebildet wird.

8. Verfahren nach einem der Ansprüche 1 bis 7, dadurch gekennzeichnet, daß man 1 Mol Keton mit 1 bis 2 Mol organischem Halogenid und 1 bis 3 gAtom Mangan umsetzt, wobei die bevorzugte Ketonkonzentration im Reaktionsmedium 0,3 bis 2 M und insbesondere 0,5 bis 1,6 M ist.

9. Verfahren nach einem der vorstehenden Ansprüche, dadurch gekennzeichnet, daß das Reaktionsmedium eine Verbindung eines Metalls der Gruppe II bis VIII des Periodensystems der Elemente, das weniger elektropositiv als Mangan ist, enthält.

10. Verfahren nach Anspruch 9, dadurch gekennzeichnet, daß die Metallverbindung ein zumindest teilweise im Reaktionsmedium gelöstes Salz von Zink, Cadmium, Zinn oder Quecksilber ist, hauptsächlich ein Halogenid, insbesondere ein Chlorid, Bromid oder Iodid, und insbesondere ein Zinksalz, vorzugsweise das Chlorid.

11. Verfahren nach Anspruch 9 oder 10, dadurch gekennzeichnet, daß man 1 Mol Aldehyd oder Keton mit 1 bis 2 Mol organischem Halogenid, 1 bis 3 gAtom Mangan und 0,01 bis 1 Mol, vorzugsweise 0,05 bis 0,1 Mol Metallsalz umsetzt, wobei die bevorzugte Konzentration an Carbonylverbindung im Reaktionsmedium 0,3 bis 2 M und insbesondere 0,5 bis 1,6 M ist.

12. Verfahren nach einem der vorstehenden Ansprüche, dadurch gekennzeichnet, daß die Reaktion zwischen der Carbonylverbindung, dem organischen Halogenid und dem Mangan zwischen 20 und 100°C, vozugsweise zwischen 30 und 60°C, stattfindet.

13. Verfahren nach einem der vorstehenden Ansprüche, dadurch gekennzeichnet, daß die Carbonylverbindung ein Keton vom Typ R-CO-$R^1$ ist, worin R und $R^1$ aliphatische Kohlenwasserstoffgruppen von vorzugsweise $C_1$ bis $C_8$, cycloaliphatische Kohlenwasserstoffgruppen von insbesondere $C_4$ bis $C_8$ oder arylische Kohlenwasserstoffgruppen, insbesondere Phenyle oder Naphthyle, sind, die Alkyle, Alkenyle oder verschiedene funktionelle Gruppen, insbesondere Ester, Amid, Nitril oder Halogen, als Substituenten tragen können.

14. Verfahren nach einem der Ansprüche 9 bis 12, dadurch gekennzeichnet, daß die Carbonylverbindung ein Aldehyd vom Typ R-CHO ist, worin R die gleiche Bedeutung wie in Anspruch 13 hat.

15. Verfahren nach einem der vorstehenden Ansprüche, dadurch gekennzeichnet, daß das organische Halogenid eine Verbindung X-$R^2$-Y ist, worin X ein Halogen ist, $R^2$ eine Kohlenwasserstoffgruppe darstellt und Y, dessen Gegenwart nicht zwingend ist, eine funktionelle Gruppe oder eine Gruppe mit einer Doppel- oder Dreifachbindung ist.

## Claims

1. Method of preparing alcohols by reacting an organic halide with a carbonyl compound, in an organic solvent, in the presence of metallic manganese, and then hydrolysing the product of the reaction, characterised in that the reaction is initiated and activated by an ester present in the reaction medium.

2. Method according to claim 1, characterised in that the total number of carbon atoms in the ester is 3 to 13, the carboxylic acid in this ester being $C_2$ to $C_{12}$ and the alcohol residue respectively $C_{12}$ to $C_1$.

3. Method according to claim 2, characterised in that the ester comes from a $C_2$ to $C_4$ aliphatic acid and a $C_1$ to $C_3$ alcohol.

4. Method according to one of claims 1 to 3, characterised in that the ester is a methyl, ethyl, propyl or isopropyl propionate, butyrate or isobutyrate.

5. Method according to one of claims 1 to 3, characterised in that the ester is ethyl acetate.

6. Method according to one of the preceding claims, characterised in that the solvent used is a conventional solvent with 5 to 90% and preferably 50 to 90% by weight of ester added.

7. Method according to one of claims 1 to 5, characterised in that the solvent consists entirely of ester.

8. Method according to one of claims 1 to 7, characterised in that 1 mole of ketone is reacted with 1 to 2 moles of organic halide and 1 to 3 gram atoms of manganese, the preferred concentration of ketone in the reaction medium being 0.3 to 2 M and especially 0.5 to 1.6 M.

9. Method according to one of the preceding claims, characterised in that the reaction medium contains a compound of a metal in group II to VIII of the Periodic Table, less electropositive than manganese.

10. Method according to claim 9, characterised in that the metal compound is a zinc, cadmium, tin or mercury salt, dissolved at least partially in the reaction medium, principally a halogen, in particular a chloride, bromide, or iodide, and notably a zinc salt, preferably chloride.

11. Method according to claim 9 or 10, characterised in that 1 mole of aldehyde or ketone is reacted with 1 to 2 moles of organic halide, 1 to 3 gram atoms of manganese and 0.01 to 1 mole, preferably 0.05 to 0.1 mole, of metallic salt, the preferred concentration of carbonyl compound in the reaction medium being 0.3 to 2 M and especially 0.5 to 1.6 M.

12. Method according to one of the preceding claims, characterised in that the reaction between the carbonyl compound, the organic halide and the manganese takes place between 20° and 100°C and preferably between 30° and 60°C.

13. Method according to one of the preceding claims, characterised in that the carbonyl compound is a ketone of the type R-CO-$R^1$, where R and $R^1$ are aliphatic hydrocarbon groups, preferably $C_1$ to $C_8$, cycloaliphatic, especially $C_4$ to $C_8$, or aryl, notably phenyls or naphthyls, able to carry alkyl or alkenyl substituents or various functional groups, in particular ester, amide, nitrile or halide.

14. Method according to one of claims 9 to 12, characterised in that the carbonyl compound is an aldehyde of the type R-CHO, R having the same signification as in claim 13.

15. Method according to one of the preceding claims, characterised in that the organic halide is a compound X-$R^2$-Y, X being a halogen, $R^2$ representing a hydrocarbon group and Y, whose presence is not obligatory, is a functional group or a group with a double or triple bond.